# FASCICULE DE BREVET EUROPEEN

(11) **EP 3 525 889 B1**
(45) Date de publication et mention de la délivrance du brevet: **02.12.2020**
(21) Numéro de dépôt: 17794420.4
(22) Date de dépôt: 13.10.2017
(51) Int. Cl.: A61Q 19/08, A61K 8/97, A61K 8/9783

(54) **EXTRAIT D'ANIGOZANTHOS FLAVIDUS POUR SON UTILISATION COSMÉTIQUE**
EXTRAKT VON ANIGOZANTHOS FLAVIDUS ZUR KOSMETISCHEN VERWENDUNG
EXTRACT OF ANIGOZANTHOS FLAVIDUS FOR COSMETIC USE

(30) Priorité: 17.10.2016 FR 1660038
(43) Date de publication de la demande: 21.08.2019
(73) Titulaire: International Flavors & Fragrances Inc., New York, NY 10019 (US)
(72) Inventeur: ATTIA, Joan, 31600 Saint Clar de Riviere (FR); SHORTT, Martin Peter, Ballina, New South Wales 2478 (AU); BEGIN-LAVALLEE, Valérie, Ville de Québec, Québec G1S 3N1 (CA); LOING, Estelle, Ville de Québec, Québec G1S 2R9 (CA)
(74) Mandataire: Cabinet Becker et Associés
(86) Numéro de dépôt international: PCT/IB2017/056370
(87) Numéro de publication internationale: WO 2018/073714

(56) Documents cités:
- DATABASE GNPD [Online] MINTEL; 1 décembre 2008 (2008-12-01), "Longevity Serum", XP002771360, Database accession no. 1014246
- DATABASE GNPD [Online] MINTEL; 1 août 2012 (2012-08-01), "Moisturising Mask", XP002771361, Database accession no. 1864866
- DATABASE GNPD [Online] MINTEL; 1 décembre 2012 (2012-12-01), "Moisturising Cleaning Foam", XP002771362, Database accession no. 1939010
- DATABASE GNPD [Online] MINTEL; 1 mars 2015 (2015-03-01), "Moisturising Gel", XP002771363, Database accession no. 3092061
- Lucas Meyer: "SouthernCross Botanicals", , 19 novembre 2014 (2014-11-19), pages 1-20, XP055384495, Extrait de l'Internet: URL:https://www.in-cosmetics.com/__novadoc uments/245955?v=636017775273900000 [extrait le 2017-06-23]
- HOLSCHER D ET AL: "HPLC-NMR analysis of phenylphenalenones and a stilbene from Anigozanthos flavidus", PHYTOCHEMI, PERGAMON PRESS, GB, vol. 50, no. 1, 15 janvier 1999 (1999-01-15), pages 155-161, XP004290668, ISSN: 0031-9422, DOI: 10.1016/S0031-9422(98)00495-6

## Description

### Domaine de l'invention

La présente invention se rapporte au domaine cosmétique, en particulier aux agents cosmétiques antiâges.

### Contexte de l'invention

La peau est la première barrière protégeant l'organisme des agressions extérieures. Cet organe est composé par plusieurs couches de tissu. On distingue l'épiderme qui est la partie la plus externe de la peau, le derme, un tissu conjonctif constitué de fibroblastes et d'une matrice extracellulaire, qui assure les fonctions de cohésion et de nutrition de la peau, et l'hypoderme constitué d'adipocytes.
L'épiderme est constitué par plusieurs strates cellulaires de kératinocytes. On distingue, entre autres, la couche germinative de l'épiderme, appelée couche basale, contenant, notamment, les cellules souches cutanées, la couche épineuse, *Stratum spinosum,* constituée de plusieurs couches de cellules polygonales, la couche granuleuse, *Stratum granulosum,* comprenant une à trois couches de cellules aplaties contenant des inclusions cytoplasmiques, les grains de kératohyaline, et enfin, le couche cornée, *Stratum corneum* qui est composée de cellules anucléés et riches en kératine appelées cornéocytes qui correspondent au stade terminal de différenciation des kératinocytes.
Les cellules les plus externes de la couche cornée sont continuellement éliminées et remplacées par les cellules d'une couche inférieure, selon un processus appelé desquamation. La régénération cellulaire de la couche cornée est basée sur un processus de maturation cellulaire dans lequel les cellules de la couche basale de l'épiderme se différencient et migrent progressivement à travers les différentes strates de l'épiderme jusqu'à arriver à la couche cornée sous la forme de cornéocytes.
Le vieillissement cutané, qu'il résulte d'un phénomène normal de sénescence ou qu'il soit accentué par un facteur extérieur tel que l'exposition aux radiations UV, implique des dysfonctionnements de la différenciation et/ou du renouvellement cellulaire se traduisant par une atrophie de l'ensemble des assises de la peau. D'un point de vue histologique, on observe, entre autres, une diminution de la qualité du derme, en particulier une diminution de l'épaisseur de l'épiderme et une perte de consistance de la matrice extracellulaire. On observe ainsi au niveau de la matrice extracellulaire que les fibres de collagène deviennent moins nombreuses, et moins régulières et que le rapport collagène de type III sur collagène de type I est augmenté. Le nombre de fibres élastiques du derme papillaire est également diminué et les fibres élastiques du derme réticulaire sont altérées. D'un point de vue esthétique, ces altérations se traduisent par une modification de l'aspect de la peau et de ses propriétés mécaniques : la peau est moins lisse, voire rugueuse, son microrelief est plus marqué, et peut présenter des ridules, pouvant conduire avec le temps à la formation de rides profondes. La peau présente également une perte d'élasticité, de tonus et de fermeté, pouvant conduire à un affaissement ou un relâchement tissulaire et une accentuation des rides, en particulier au niveau du visage et du cou. Le vieillissement cutané peut avoir un impact sur l'homogénéité du teint et entraîner l'apparition de tâches pigmentaires.
Il existe sur le marché de nombreux produits cosmétiques antiâges destinés à prévenir ou à atténuer le relâchement de la peau.
La base Mintel décrit un sérum antiâge commercialisé par la société La Prairie comprenant plusieurs extraits végétaux, dont un extrait d'*Anigozanthos flavidus* (N° d'enregistrement sur la base Mintel : 1014246).
Néanmoins, il reste, à l'heure actuelle, un besoin pour de nouveaux actifs antiâges pour prévenir ou traiter des altérations résultant du vieillissement cutané, en particulier liées au relâchement cutané.

### Résumé de l'invention

L'invention a pour objet l'utilisation cosmétique d'un extrait d'une partie aérienne d'*Anigozanthos flavidus* en tant qu'agent antiâge. En particulier, l'extrait d'*Anigozanthos flavidus* peut être utilisé en tant qu'agent anti-ride, lissant, tonifiant, restructurant ou remodelant de la peau.
Typiquement, l'extrait d'*Anigozanthos flavidus* est présent en tant qu'agent actif dans une composition cosmétique destinée à prévenir, retarder et/ou traiter un ou plusieurs signes du vieillissement cutané. Le ou les signes du vieillissement cutané peuvent être choisis parmi le groupe constitué par : l'apparition de ridules et/ou de rides sur la peau, notamment au niveau des lèvres et des paupières, un flétrissement ou un affaissement de la peau, notamment au niveau du cou, une perte de densité de la peau, une perte de fermeté de la peau, une perte de tonicité de la peau, une perte d'élasticité de la peau, une perte de contractilité de la peau, une perte de maintien de la peau, un relâchement de la peau, une altération de l'aspect lisse de la peau, une augmentation de la rugosité de la peau, et une altération du contour du visage.

L'extrait *d'Anigozanthos flavidus* est obtenu à partir d'une partie aérienne *d'Anigozanthus flavidus,* par exemple les fleurs, les feuilles, les tiges et leurs combinaisons. Dans certains modes de réalisation, l'extrait d'*Anigozanthos flavidus* est obtenu par extraction avec un solvant polaire, de préférence à chaud.

Comme mentionné ci-avant, l'extrait d'*Anigozanthos flavidus* peut être présent en tant qu'actif dans une composition cosmétique. Ladite composition cosmétique peut comprendre au moins un agent cosmétique additionnel, de préférence choisi dans le groupe constitué par les vitamines, les filtres et écrans solaires, les agents anti-âges, ou antirides, les antioxydants, les agents liftants, les agents raffermissants, les agents anti-tâches, les agents anti-rougeurs, les agents amincissants, les agents drainants, les agents hydratants, les agents apaisants, les agents gommants ou exfoliants, les agents matifiants, les agents séborégulateurs, les actifs éclaircissants, les actifs auto-bronzants, les accélérateurs de bronzage et leurs combinaisons. Dans certains modes de réalisation, la composition cosmétique est choisie parmi le groupe constitué par les solutions aqueuses, les solutions hydroalcooliques, les émulsions huile-dans-eau (H/E) ou eau-dans huile (E/H) ou multiple (triple : E/H/E ou H/E/H), les nanoémulsions, en particulier des nanoémulsions H/E, dont la taille des gouttes est inférieure à 100nm, les gels aqueux, ou les dispersions d'une phase grasse dans une phase aqueuse à l'aide de sphérules, les suspensions, de préférence en milieux aqueux ou hydroalcoolique, les suspensions de liposomes, les poudres, les lotions, laits, les crèmes, les onguents, les gels, les mousses, et les pommades. La composition cosmétique peut être choisie parmi un produit cosmétique, un produit de maquillage ou un produit d'hygiène corporelle, par exemple une lotion, un lait, un sérum, un gel aqueux ou huileux, une émulsion, une crème notamment une crème de jour ou une crème de nuit, un gel-crème, une eau de soin, une pommade, un baume, un fond de teint, un spray, un ombre à paupière, un stick, un rouge à lèvre, un gloss, un baume à lèvres, une mousse, un déodorant, un masque nourrissant, un gel douche, et un produit exfoliant ou gommant. Divulguée ici est également une composition cosmétique pour une utilisation dans la prévention ou le traitement d'un signe du vieillissement, ladite composition comprenant un extrait d'*Anigozanthos flavidus,* notamment tel que défini ci-avant et un ou plusieurs excipients acceptables sur le plan pharmaceutique. Selon un aspect supplémentaire, la dite composition est une composition « précurseur », c'est-à-dire une composition destinée à la préparation d'une composition cosmétique comprenant :
- de 10% à 50%, de préférence de 20% à 40% d'un extrait d'*Anigozanthos flavidus,* de préférence un extrait liquide *d'Anigozanthos flavidus,*
- de 50% à 90%, de préférence de 60% à 80% d'un véhicule, de préférence choisi parmi un solvant aqueux, un solvant organique, de préférence un alcool inférieur tel que l'éthanol, le propanediol, le butylène glycol, le glycérol ou l'isopropanol, un agent lipophile et leurs mélanges, et
- en option, de 0,1 à 30% d'un excipient supplémentaire acceptable sur le plan cosmétique, de préférence choisi parmi un agent ajusteur de pH, un agent antioxydant, un agent conservateur, un agent stabilisant, un agent de vectorisation, un agent épaississant, un émulsifiant, un agent gélifiant hydrophile ou lipophile, un parfum, une huile minérale ou organique, et leurs combinaisons.
les pourcentages étant exprimés en poids par rapport au poids total de ladite composition. Divulguée ici est aussi une composition pour la préparation d'une composition cosmétique comprenant :
- de 20% à 40 % d'un extrait aqueux obtenu à partir d'une partie aérienne *d'Anigozanthos flavidus,* et
- de 60% à 80% de glycérol.
les pourcentages en poids étant exprimés par rapport au poids total de ladite composition. Divulgué ici est également un procédé de préparation d'une composition cosmétique selon l'invention comprenant une étape de mélange d'une composition « précurseur » selon l'invention avec un ou plusieurs excipients sur le plan cosmétique et/ou avec un ou plusieurs actifs additionnels à effet cosmétique.

### Présentation des figures

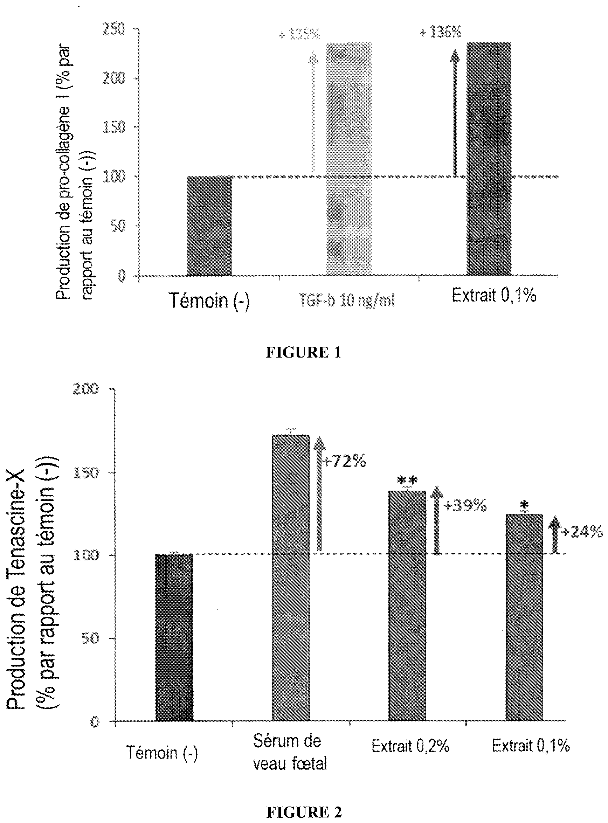

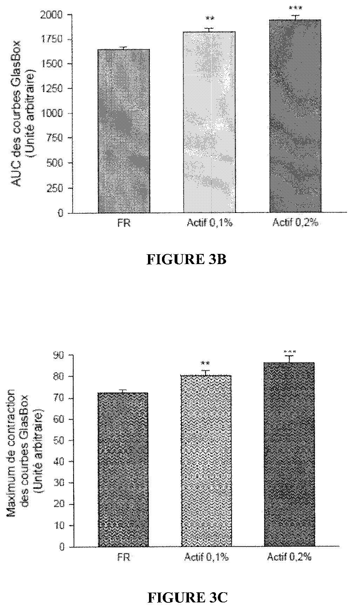

La **Figure 1** montre la quantité de pro-collagène I exprimée en pourcentage par rapport au Témoin produite par des fibroblastes incubés avec le TGF-β ou avec une concentration de 0,1% de la composition dénommée « Extrait » (Exemple 1). La composition « Extrait » comprend 5% en poids d'un extrait aqueux de fleurs d'*Anigozanthos flavidus,* moins de 35% en poids d'eau, et plus de 50% en poids de glycérine.
La **Figure 2** montre la quantité de Tenascine-X exprimée en pourcentage par rapport au Témoin produite pas des fibroblastes incubés avec du sérum de veau fœtal (SVF), avec une concentration de 0,2% de la composition « Extrait » (Extrait 0,2%) et en présence d'une concentration de 0,1% de la composition « Extrait » (Extrait 0,1%) (Exemple 2).

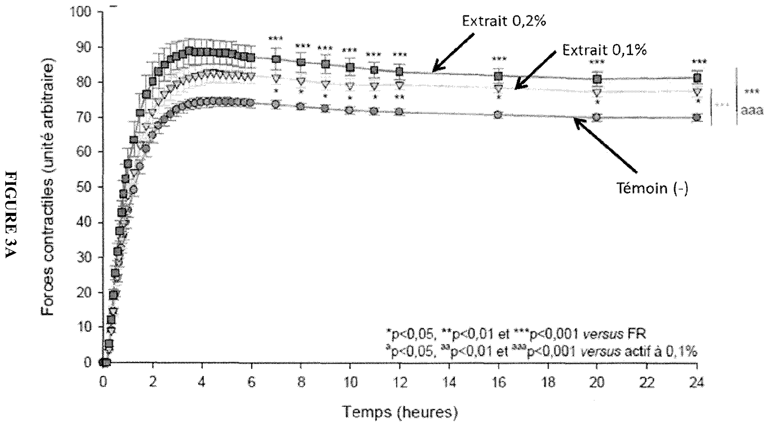

Les **Figures 3A-C** présentent les résultats relatifs à l'effet de l' « Extrait » sur les forces contractiles des fibroblastes humains de fond de ride (FR) (Exemple 4). La **Figure 3A** montre l'évolution des forces contractiles appliquées par les fibroblastes de FR en présence de 0,1% (v/v) de la composition « Extrait », en présence de 0,2% (v/v) de la composition « Extrait », et en l'absence d'actif (Témoin (-)), pendant 24h.
Les histogrammes de la **Figure 3B** correspondent aux aires sous courbe (AUC) des courbes de la Figure 3A. Les histogrammes de la **Figure 3C** correspondent aux forces maximales de contraction pour les courbes de la Figure 3A.
   FR : Fibroblaste FR sans actif (témoin négatif), Fibroblastes FR incubés en présence de 0,1% (v/v) de la composition « Extrait » (Actif 0.1%), et de 0,2% (v/v) la composition « Extrait » (Actif 0.2%)
La **Figure 4** montre les clichés d'immuno-marquage des fibres α-SM actine au niveau des fibroblastes de fond de ride témoins (A), après incubation avec 0,2% d' « Extrait » (B) et après incubation avec 0,1% d' « Extrait » (C).
Les **Figures 5A et 5B** montrent les forces contractiles exercées par le derme équivalent acellulaire en fonction du temps en présence de la composition « Extrait » (Actif à 0,2%) (Kangaroo Paw 0.2%) et en l'absence de ladite composition (Témoin).
La **Figure 6** montre la variation de la profondeur des rides de la patte d'oie du visage traitées par une crème placebo (crème A) en comparaison avec une crème contenant l'« Extrait » à 2% (crème B), 1h après l'application de l'Extrait et après 28 jours de traitement.

### Description de l'invention

*Anigozanthos flavidus* est une espèce végétale endémique de la famille des Haemodoraceae que l'on rencontre principalement dans le sud-ouest de l'Australie. Cette plante se caractérise par une fleur, généralement de couleur jaune ou rouge dont la forme évoque la patte d'un kangourou. A la connaissance de la Demanderesse, les extraits d'*Anigozanthos flavidus* ont été utilisés en cosmétique, tout au plus, en tant qu'agent conditionneur, par exemple, dans des compositions de shampooing. Aucune activité biologique cosmétique ne semble avoir été décrite pour les extraits *d'Anigozanthos flavidus.*
La Demanderesse a préparé un extrait aqueux *d'Anigozanthos flavidus* et a étudié les activités biologiques de cet extrait.
De manière surprenante, la Demanderesse a montré que l'extrait d'*Anigozanthos flavidus* était actif sur des cibles biologiques jouant un rôle clé dans l'organisation et la composition de la matrice extracellulaire de la peau. Comme cela est illustré dans les exemples, l'extrait d'*Anigozanthos flavidus* est capable de stimuler la synthèse de pro-collagène I par les fibroblastes du derme et d'augmenter la synthèse de collagène XVII par les kératinocytes. La Demanderesse a également montré que l'extrait d'*Anigozanthos flavidus* est capable de stimuler la synthèse de tenascine-X, une glycoprotéine jouant un rôle-clé dans la structure et la stabilité des fibres élastiques et dans l'organisation des fibres de collagènes de la matrice extracellulaire de la peau. L'extrait *d'Anigozanthos flavidus* stimule également la synthèse d'alpha-actin 2 (aussi connue sous le nom de l'alpha smooth muscle actin (α-SMA)) chez les fibroblastes ce qui a pour conséquence d'augmenter les forces contractiles développées par les fibroblastes issus de fond de rides. Il a été également montré que l'extrait *d'Anigozanthos flavidus* était capable d'augmenter les forces contractiles exercées par un derme équivalent acellulaire, ce qui suggère un effet tenseur immédiat de l'extrait sur les fibres du derme par une action physique.
La Demanderesse a également réalisé des tests cliniques visant à évaluer les effets antiâges d'une crème de jour comprenant 0,6% en poids d'un extrait aqueux d'*Anigozanthos flavidus.* Ces essais ont montré que l'extrait *d'Anigozanthos flavidus* exerce un effet tenseur-remodelant au niveau du visage en atténuant de manière significative la profondeur des rides sur le long terme. Il a été également montré que l'extrait d'*Anigozanthos flavidus* exerce un effet tenseur immédiat, observable dès les premières minutes après son application sur la peau.
Au regard de ces résultats, les extraits d'*Anigozanthos flavidus* trouvent donc des applications dans le domaine cosmétique en tant qu'agents antiâges pour prévenir ou traiter les signes du vieillissement cutané. Les extraits d'*Anigozanthos flavidus* trouvent également des applications en tant qu'agents tenseurs à action immédiate afin de retendre la peau et/ou d'améliorer la tenue ou le maintien de la peau de la peau, à court terme.
Grâce à leurs actions sur la synthèse de plusieurs protéines clés impliquées dans l'organisation, la cohésion et l'élasticité de la matrice extracellulaire de la peau, les extraits d'*Anigozanthos flavidus* peuvent être utilisés pour promouvoir la restructuration et la réorganisation de la matrice extracellulaire, en particulier, du derme chez une peau mature ou âgée. De manière générale, les extraits d'*Anigozanthos flavidus* peuvent être utilisés pour traiter ou prévenir le relâchement cutané, améliorer l'élasticité de la peau, ou encore traiter ou prévenir les rides et ridules de la peau. Ainsi, les extraits d'*Anigozanthos flavidus* peuvent être utilisés en tant qu'agents tenseurs ou liftant ou en tant qu'agents remodelant et restructurant de la peau. En particulier, les extraits d'*Anigozanthos flavidus* peuvent être utilisés en tant qu'agent cosmétique pour traiter ou prévenir une altération non-pathologique de la peau liée à une perte d'élasticité de la peau et/ou à une altération de la matrice extracellulaire de la peau, par exemple une désorganisation de la matrice extracellulaire résultant d'une altération qualitative ou quantitative des constituants de la matrice extracellulaire tels que les fibres élastiques.

### ▪ Utilisations de l'extrait d'Anigozanthos flavidus selon l'invention

Selon un premier aspect, la présente invention concerne l'utilisation cosmétique, non thérapeutique, d'un extrait d'*Anigozanthos flavidus.* L'extrait d'*Anigozanthos flavidus* peut être utilisé en tant qu'agent cosmétique, notamment en tant qu'agent antiâge.
Dans le cadre de la présente demande, un extrait d'*Anigozanthos flavidus* se réfère à un extrait obtenu à partir d'une partie aérienne de la plante *Anigozanthos flavidus* telle que les feuilles, les fleurs, les tiges et leurs combinaisons. Dans un mode de réalisation particulier, l'extrait d'*Anigozanthos flavidus* est un extrait obtenu à partir des fleurs et/ou des tiges *d'Anigozanthos flavidus.* L'extrait d'*Anigozanthos flavidus* peut être obtenu par toute technique connue d'extraction. De préférence, l'extrait d'*Anigozanthos flavidus* est préparé par extraction à l'aide d'un solvant polaire. Typiquement, le solvant polaire peut être choisi parmi l'eau, un alcool, un ester, une cétone et leurs mélanges. On peut citer, à titre d'exemple d'esters, l'acétate d'éthyle, l'acétate de propyle et l'acétate de butyle. Les alcools englobent, sans y être limités, le méthanol, l'éthanol, le n-propanol, l'isopropanol, le méthanol, et les polyols tels que le glycérol. On peut citer, à titre d'exemple de cétones, l'acétone, la cyclohexanone ou encore la méthyl éthyl cétone.
Dans un mode de réalisation particulier, le solvant d'extraction est choisi parmi l'eau, un alcool et un mélange hydro-alcoolique, par exemple un mélange eau/éthanol, eau/méthanol ou eau/glycérol. Dans certains modes de réalisation particulière, le solvant d'extraction est l'eau.
L'extraction par le solvant polaire peut être réalisée par toute méthode connue de l'homme du métier, par exemple par décoction, infusion, macération, percolation ou lixiviation. Par exemple, l'extrait d'*Anigozanthos flavidus* peut être obtenu par extraction à chaud dans l'eau ou dans un mélange hydro-alcoolique. Une technique adaptée d'extraction à chaud est la décoction.
On entend par « décoction », le fait de faire bouillir une matière végétale dans de l'eau pendant une durée adaptée pour obtenir une extraction efficace.
L'étape d'extraction peut être réalisée sur une matière végétale *d'Anigozanthos flavidus* ayant subi un ou plusieurs traitements tels qu'une congélation, un séchage, une lyophilisation, un broyage et les combinaisons de ceux-ci. Dans certains modes de réalisation, l'extraction est réalisée sur une matière végétale fraîche ou séchée ayant subi une étape de broyage. A l'issue de l'extraction, l'extrait peut être récupéré par toute méthode permettant de séparer la matière végétale et la phase liquide, par exemple par centrifugation et/ou filtration.
A titre d'exemple, l'extrait d'*Anigozanthos flavidus* est susceptible d'être obtenu par un procédé comprenant :
- Une étape de préparation d'une matrice végétale par séchage et broyage d'une partie aérienne, typiquement des fleurs et des tiges, d'*Anigozanthos flavidus*
- une étape d'extraction de la matière végétale par un solvant polaire, typiquement l'eau ou un mélange hydroalcoolique, de préférence à chaud, par exemple par décoction, et
- une étape d'élimination de la matrice végétale solide, de préférence par filtration.
Le procédé de préparation de l'extrait d'*Anigozanthos flavidus* peut comprendre une ou plusieurs étapes supplémentaires à l'issue de l'étape d'extraction et/ou de filtration. Le procédé peut comprendre une étape de formulation de l'extrait, par exemple par ajout d'un agent pour ajuster le pH, d'un agent stabilisant et/ou d'un véhicule ou support cosmétiquement acceptable, une étape de concentration de l'extrait, ou encore une étape de séchage de l'extrait.
Dans un mode de réalisation supplémentaire ou alternatif, l'extrait *d'Anigozanthos flavidus* comprend des flavonoïdes, des polyphénols et des glucides. Les flavonoïdes peuvent représenter de 1% à 15%, par exemple de 5% à 15% ou 1% à 8%en équivalence naringine par rapport à la matière sèche de l'extrait ; les polyphénols peuvent représenter de 5% à 30% par exemple de 5% à 25%, de 12% à 20%, de 10% à 30%, ou de 15% à 20%, en équivalence acide chlorogénique en poids par rapport à la matière sèche de l'extrait ; et les sucres peuvent représenter de 35% à 65%, par exemple de 35% à 55%, de 40% à 60% ou de 40% à 50%, en équivalence glucose en poids par rapport à la matière sèche de l'extrait. Les teneurs en polyphénols, flavonoïdes et sucres sont déterminées par colorimétrie en utilisant une gamme étalon obtenue à partir de l'acide chlorogénique (Méthode de Folin-Ciocalteu), la naringénine (Méthode de Zhishen et al., Food Chemistry, 1999, 64, 555-559) et le glucose (Méthode de Bachelier et al., Cah. OSTRUM.sér. Pédol. Vo. IV,3-1966) respectivement. L'extrait d'*Anigozanthos flavidus* obtenu à l'issue du procédé d'extraction peut comprendre une teneur en matière sèche allant de 0,1 % à 40% en poids, de préférence de 0,1% à 10% en poids, par exemple de 0,1% à 4% en poids.

Dans certains modes de réalisation, l'extrait d'*Anigozanthos flavidus* comprend moins de 1% en poids d'acide férulique par rapport à la matière sèche de l'extrait. De préférence, l'extrait d'*Anigozanthos flavidus* est dépourvu d'acide férulique.
Dans un mode de réalisation particulier, l'extrait d'*Anigozanthos flavidus* est caractérisé par une ou plusieurs (e.g. 2, 3, 4, 5 voire toutes) caractéristiques suivantes :
- L'extrait d'*Anigozanthos flavidus* est obtenu à partir d'une partie aérienne d'*Anigozanthos flavidus* par extraction à l'aide d'un solvant polaire, de préférence par décoction dans l'eau ou dans un mélange hydroalcoolique,
- L'extrait d'*Anigozanthos flavidus* comprend une teneur en matière sèche de 0,1% à 10% en poids,
- Les flavonoïdes représentent de 5% à 15%, par exemple de 7% à 13%, en équivalence naringine en poids par rapport à la matière sèche de l'extrait,
- Les sucres représentent de de 35% à 65%, par exemple de 40% à 60% en équivalence glucose en poids par rapport à la matière sèche de l'extrait,
- Les polyphénols représentent de 5% à 25% par exemple de 10% à 20%, en équivalence acide chlorogénique en poids par rapport à la matière sèche de l'extrait, et/ou
- L'extrait d'*Anigozanthos flavidus* comprend moins de 1% en poids d'acide férulique par rapport à la matière sèche de l'extrait.
Dans les utilisations selon l'invention, l'extrait *d'Anigozanthosflavidus* peut être sous forme liquide, par exemple dans l'eau ou dans un mélange hydroalcoolique tel qu'un mélange eau/glycérol. Dans d'autres modes de réalisation, l'extrait d'*Anigozanthos flavidus* peut être sous forme solide sèche, typiquement sous la forme d'une poudre. Un extrait sec d'*Anigozanthos flavidus* peut être obtenu en éliminant le solvant d'extraction par toute technique connue, par exemple par atomisation, évaporation ou lyophilisation.
A titre d'exemple supplémentaire, l'extrait d'*Anigozanthos flavidus* peut être sous une forme absorbée sur un véhicule solide tel que la maltodextrine.
Dans certains modes de réalisation, l'extrait d'*Anigozanthos flavidus* est un extrait liquide dans l'eau ou dans un mélange hydroalcoolique, de préférence dans un mélange eau/glycérine. Ledit mélange hydroalcoolique peut présenter un rapport volumique eau/alcool allant de 1/9 à 9/1, de préférence de 1/9 à 2/1.
Au sens de l'invention, on entend par « *agent antiâge* », un agent actif cosmétique ayant une activité biologique permettant de prévenir, ou traiter un signe du vieillissement cutané.

On entend par « *prévenir un signe du vieillissement cutané* », le fait de retarder ou d'empêcher l'apparition du signe du vieillissement cutané.
On entend par « *traiter un signe du vieillissement cutané* », le fait de diminuer, d'atténuer, d'estomper, de corriger ou ralentir le développement du signe du vieillissement cutané.
On entend par « *un signe du vieillissement cutané* » toute altération ou modification de l'aspect visuel ou des propriétés mécaniques de la peau, non-pathologique, résultant du vieillissement, qu'il soit chronologique (chrono-vieillissement) et/ou du photo-induit (photo-vieillissement). Un signe du vieillissement cutané se réfère donc à une altération ou modification non-pathologique de l'aspect ou des propriétés mécaniques de la peau.
Ainsi, les signes du vieillissement englobent, sans y être limités, un amincissement de la peau, en particulier de l'épiderme, l'apparition d'un microrelief, l'apparition de ridules et/ou de rides sur la peau, y compris au niveau des lèvres et des paupières, un flétrissement ou un affaissement de la peau, une perte d'éclat de la peau, un teint brouillé, les tâches pigmentaires cutanées, les cernes au niveau des yeux, une perte de densité de la peau, une perte de fermeté de la peau, une perte de tonicité de la peau, une perte de contractilité de la peau, une perte d'élasticité de la peau, une altération de l'aspect lisse de la peau, une augmentation de la rugosité de la peau et une altération du contour du visage. Au sens de l'invention, les termes *« peau »* et « *cutané* » se réfère à toute partie de la peau du corps humain, en particulier la peau du visage, y compris les lèvres et les paupières, le cou, et la peau des mains. Comme présenté ci-avant, l'extrait *d'Anigozanthos flavidus* est capable de promouvoir la synthèse de différentes protéines clés impliquées dans la structure de la matrice extracellulaire et dans l'élasticité de la peau.
Ainsi, l'extrait *d'Anigozanthos flavidus* peut être utilisé pour améliorer, restaurer, ou prévenir l'altération des propriétés mécaniques de la peau, notamment la souplesse, l'élasticité, la fermeté ou la tonicité de la peau. Par exemple, l'extrait d'*Anigozanthos flavidus* peut être utilisé pour améliorer, restaurer, ou prévenir l'altération des propriétés mécaniques d'une peau mature ou d'une peau âgée.
On entend par « *peau mature* », la peau d'un homme ou d'une femme âgée de plus de 40 ans et présentant une perte d'élasticité due au vieillissement.
Dans un mode de réalisation particulier, l'extrait selon l'invention est utilisé pour traiter ou prévenir un signe du vieillissement cutané associé à une altération des propriétés mécaniques de la peau, notamment une perte d'élasticité de la peau. L'extrait d'*Anigozanthos flavidus* peut être ainsi utilisé en tant qu'agent lissant, défroissant, liftant ou reliftant, restructurant ou remodelant de la peau. L'extrait d'*Anigozanthos flavidus* peut être également utilisé en tant qu'agent tenseur immédiat de la peau.
On entend par « *un agent lissant ou défroissant»* un agent cosmétique capable d'atténuer et/ou de corriger le microrelief de la peau, y compris des lèvres, ledit microrelief se présentant sous la forme de rides ou de ridules.
On entend par « *agent liftant ou reliftant* » un agent cosmétique capable d'exercer une action de tenseur sur la peau ce par quoi l'aspect de la peau est amélioré et les rides moins visibles. On entend par « *agent remodelant de la peau »* un agent capable d'atténuer l'aspect irrégulier de la peau consécutif à une perte d'élasticité ou un relâchement cutané, notamment au niveau du contour du visage et du cou.
On entend par « *agent liftant* » un agent cosmétique capable d'exercer un effet tenseur sur la peau de manière à estomper le microrelief de la peau.
On entend par « *agent restructurant »* un agent cosmétique capable de prévenir ou traiter une altération de la matrice extracellulaire de la peau résultant du vieillissement, telle qu'une perte ou une altération des fibres élastiques de la matrice extracellulaire ou encore une perte ou une altération du collagène de la matrice extracellulaire.
On entend par « *agent tenseur immédiat ou agent tenseur à effet immédiat* » un agent cosmétique capable d'exercer un effet tenseur sur la peau de manière à augmenter l'élasticité et/ou améliorer la tenue ou le maintien de la peau, cet effet pouvant être visible typiquement entre quelques minutes (par exemple 5 minutes) et une heure après application sur la peau. A titre d'exemple, l'extrait d'*Anigozanthos flavidus* peut être utilisé afin de prévenir ou traiter l'apparition de ridules et/ou de rides sur la peau, notamment au niveau des lèvres et des paupières, un flétrissement cutané, par exemple au niveau du cou, un affaissement ou un relâchement cutané, une perte de densité de la peau, une perte de fermeté de la peau, une perte de tonicité de la peau, une perte d'élasticité de la peau, un relâchement de la peau, une altération de l'aspect lisse de la peau, une augmentation de la rugosité de la peau, et une altération du contour du visage. L'extrait d'*Anigozanthos flavidus* peut être utilisé également pour rendre un aspect plus jeune à une peau mature ou âgée et/ou améliorer le teint ou l'éclat d'une peau mature ou âgée.
L'extrait d'*Anigozanthos flavidus* peut être utilisé à des fins cosmétiques pour restructurer ou réorganiser la matrice extracellulaire de la peau altérée à cause du vieillissement. En particulier, l'extrait d'*Anigozanthos flavidus* peut être utilisé à des fins cosmétiques pour stimuler la synthèse d'une protéine impliquée dans l'organisation de la matrice extracellulaire de la peau. On entend par « *protéine impliquée dans l'organisation de la matrice extracellulaire de la peau »* une protéine constitutive de la matrice extracellulaire, telle que les collagènes, l'élastine ou la fibrilline, ou une protéine impliquée dans l'expression ou dans l'organisation des protéines constitutives de la matrice extracellulaire telle que la tenascine-X. Dans certains modes de réalisation, l'extrait d'*Anigozanthos flavidus* est utilisé en tant qu'agent cosmétique pour stimuler la synthèse d'une protéine choisie parmi le pro-collagène I, le collagène XVII, et la tenascine-X au niveau de la peau, de préférence au niveau d'une peau mature. Dans un autre mode de réalisation, l'extrait d'*Anigozanthos flavidus* est utilisé en tant qu'agent cosmétique pour augmenter les forces contractiles des fibroblastes dermiques d'une peau mature ou âgée. Dans un autre mode de réalisation, l'extrait *d'Anigozanthos flavidus* est utilisé pour stimuler le métabolisme du derme.
Dans certains modes de réalisation, l'extrait *d'Anigozanthos flavidus* est utilisé en combinaison avec un agent actif à effet cosmétique. De tels agents sont décrits ci-après et englobent, entres autres, les agents antirides, les agents antiâges, les agents antioxydants, les agents hydratants, les agents liftants, et les agents raffermissants.

Dans les utilisations selon l'invention, l'extrait d'*Anigozanthos flavidus* est appliqué sur la peau. Ledit extrait est de préférence présent à titre d'agent cosmétique dans une composition destinée à être appliquée sur la peau. Ladite composition est typiquement une composition cosmétique. L'extrait d'*Anigozanthos flavidus* représente généralement de 0,0001% à 10% en poids, de préférence de 0,001% à 5% en poids, de manière plus préférée de 0,005% à 3% en poids par rapport au poids total de ladite composition cosmétique. A titre d'exemple, l'extrait *d'Anigozanthos flavidus* peut représenter de 0,01% à 3,0% en poids par exemple 0,6% en poids de la composition cosmétique. L'extrait *d'Anigozanthos flavidus* peut être ajouté dans la composition cosmétique sous la forme d'un extrait liquide, sous la forme d'un extrait sec ou encore sous la forme d'une composition « précurseur » telle que décrite plus loin dans la présente description.
Ladite composition cosmétique peut comprendre un ou plusieurs excipients acceptables sur le plan cosmétique et optionnellement un ou plusieurs principes actifs additionnels à effet cosmétique. Typiquement, ladite composition cosmétique comprend :
- de 0,0001% à 10% d'extrait d'*Anigozanthos flavidus,*
- de 0% à 20% d'un ou plusieurs agents actifs additionnels, et
- de 70% à 99,9999% d'un ou plusieurs excipients acceptables sur le plan cosmétique,
   les pourcentages étant exprimés en poids par rapport au poids total de la composition cosmétique.
Dans certains modes de réalisation, ladite composition cosmétique comprend
- de 0,001% à 5% en poids d'extrait d'*Anigozanthos flavidus,*
- de 0,001% à 10% d'un ou plusieurs agents actifs additionnels, et
- de 85% à 99,998 % d'un ou plusieurs excipients acceptables sur le plan cosmétique.
Dans d'autres modes de réalisation, ladite composition cosmétique comprend :
- de 0,01% à 5% en poids d'extrait d'*Anigozanthos flavidus,*
- de 0,01% à 10% d'un ou plusieurs agents actifs additionnels, et
- de 85% à 99,98 % d'un ou plusieurs excipients acceptables sur le plan cosmétique.
L'extrait d'*Anigozanthos flavidus* est de préférence présent sous forme liquide.
Dans un mode de réalisation complémentaire ou alternatif, la composition cosmétique comprend de 0,001% à 10%, par exemple de 0,005% à 1% ou encore de 0,005% à 0,5% en poids de matière sèche issue de l'extrait d'*Anigozanthos flavidus.*
On entend par « *principe actif à effet cosmétique, agent actif à effet cosmétique, agent cosmétique ou actif à effet cosmétique* » un composé capable d'exercer au moins un effet cosmétique sur la peau ou ses annexes. On entend par « *effet cosmétique* » tout effet non-thérapeutique visant à modifier et/ou améliorer l'aspect de la peau ou des muqueuses comme les lèvres, à les protéger des agressions externes (soleil, vent, humidité, sécheresse, produits chimiques), ou encore à prévenir et/ou à corriger les phénomènes liés à leur vieillissement. Ainsi, dans certains modes de réalisation, l'extrait d'*Anigozanthos flavidus* peut être présent dans une composition cosmétique qui comprend, en outre, un principe actif additionnel à effet cosmétique. Ce principe actif à effet cosmétique peut être choisi parmi le groupe constitué par les vitamines, les filtres et écrans solaires, les agents antiâges les agents anti-rougeurs, les antioxydants, les agents raffermissants, les agents hydratants, les agents apaisants, les agents gommants ou exfoliants, les agents matifiants, les agents séborégulateurs, les actifs éclaircissants, les actifs anti-tâches, les agents amincissants, les agents drainants, les actifs auto-bronzants, les accélérateurs de bronzage et leurs combinaisons. Notamment, la composition cosmétique peut comprendre des tocophérols, et/ou des extraits de plantes comme des extraits de graines de lin, des extraits d'exopolysaccharides de *Vibrio,* des peptides comme le trifluoroacetyl tripeptide-2.
De préférence, la composition cosmétique peut comprendre un actif choisi parmi un agent antiride, un agent anti-rougeur, un agent antioxydant, un actif hydratant, un agent apaisant, un agent sébo-régulateur, un agent anti-tâche, un filtre ou un écran solaire, et leurs combinaisons. De manière encore plus préférée, l'agent actif à effet cosmétique additionnel est choisi parmi un agent antiride, un agent antioxydant, un agent hydratant, un agent raffermissant et leurs combinaisons.
A titre d'exemple d'agents hydratants, on peut citer l'urée, l'acide pidolique (PCA) et ses dérivés en particulier ses sels tels que l'arginine PCA, le chitosan PCA, ses sels de cuivre (Cuivre PCA), de magnésium (magnésium PCA), de sodium (sodium PCA) ou de zinc, l'éthylhéxyl PCA, le gluconate de calcium, l'acide hyaluronique et ses sels et autres glycosaminoglycanes, le frucose, le glucose, l'isomaltose, le lactose, le tréhalose, le polydextrose, le saccharose (Sucrose), le maltitol, le mannitol, le sorbitol, le xylitol et les autres hydrates de carbones et dérivés, les polyéthylène glycols tels que les PEG-7, PEG-8, PEG-10, PEG-12 ou PEG-14, la glycérine, le propylène glycol, le butylène glycol, la betaïne, la citrulline, le collagène et ses dérivés, l'histidine, les hydrolysats de soie, de kératine ou de soja, les extraits de plante riches en polysaccharides et/ou polyphénols, par exemple les extraits d'Aloès, bleuet (*Centaurea cyanus*), et les combinaisons de ceux-ci.
A titre d'exemple d'agents anti-âge, on peut citer l'acide ascorbique et ses dérivés comme l'ascorbyl phosphate de magnésium, les glycosaminoglycanes et leurs dérivés, les polysaccharides de *Cyathea,* le collagène, les extraits de graines de lin (*Linum usitatissimum*), les peptides comme le Caprooyl-Tetrapeptide-3 et le trifluoroacétyl tripeptide-2, les extraits de *Polygonum aviculare,* les extraits d'algues brunes, en particulier d'*Ascophyllum nodosum,* les extraits de fougères, en particulier de *Cyathea Cumingii.*
A titre d'exemple d'agents apaisants, on peut citer l'allantoïne, les extraits d'aloès, de bouleau (par exemple *Betula alba*), d'épilobe (*Epilobium angustifolium*), de châtaignier (par exemple *Castenea sativa*)*,* de bleuet (par exemple *Centaurea cyanus*), de centella (par exemple *Centella asiatica*), de prêle (par exemple *Equisetum arvense*), de fenouil (par exemple *Foeniculum vulgare*), d'hamamélis (par exemple *Hamamelis virginiana*), de lierre (par exemple *Hedera helix*), d'*habiscus sabdariffa,* de lis (par exemple *Lilium candidum*), de mauve (par exemple *Malva sylvestris*), de mélisse (par exemple *Melissa officinalis*), de scutellaire (par exemple *Scutellaria baicalensis*), de mimosa (par exemple *Mimosa tenuiflora*), de potentille (par exemple *Potentilla erecta*), un extrait d'oligosaccharides ou un oligosaccharide, par exemple de lin, les peptides comme le palmitoyl tripeptide-8, et les combinaisons de ceux-ci.
A titre d'exemple d'agents antioxydants, on peut citer le HMR (hydroxy méthyl résorcinol), l'acide ascorbique et ses dérivés, la vitamine B9, le chlorhydrate d'histidine, ou un extrait d'épilobe (*Epilobium augustifolium*). Les principes actifs à effet antioxydant et de type vitamine sont généralement utilisés en un pourcentage massique d'au moins 1% par rapport au poids total de la composition cosmétique.
A titre d'exemple d'agents séborégulateurs, on peut citer les lignanes de lin, la poudre de riz, le gluconate de zinc, la sarcosine, un extrait de *Cinnamomum zeylanicum bark,* un extrait d'avocat et les combinaisons de ceux-ci.
A titre d'agents anti-rougeurs, on peut citer les saponines, les flavonoïdes, les ruscogénines, les esculosides, et les extraits les contenant, par exemples les extraits de *Ruscus,* ainsi que certaines huiles essentielles, par exemple de lavande ou de romarin.
A titre d'exemple d'agents anti-tâches, on peut citer des extraits comme la réglisse (*Glycyrrhyza glabra*), l'extrait de jackfruit (*Artocarpus heterophyllus*), les extrait de Rumex (*R.occidentalis*), les extraits de plante appartenant au genre citrus, le resveratrol, les peptides comme l'oligopeptide-68, le nonapeptide-1, l'acide kojique, le magnésium ascorbyl phosphate et les combinaisons de ceux-ci.
Le ou les excipients acceptables sur le plan cosmétique présents dans la composition cosmétique peuvent être choisis parmi les agents diluants, les agents dispersants, les agents gélifiants, les émollients, les agents de vectorisation comme les polymères polycationiques ou les phospholipides, les gommes, les résines, les solvants en particulier les alcools inférieurs notamment l'éthanol, l'isopropanol, le dipropylène glycol, le butylène glycol, le propanediol, la glycérine, le sorbitol, et le propylène glycol, les charges telles que les amidons modifiés et polymérisés, le dioxyde de titane, ou un stéarate métallique, les conservateurs, les huiles essentielles, les agents nacrés, les colorants, les absorbeurs d'odeur, les agents régulateurs du pH ou les agents neutralisants, les agents lubrifiants, les agents épaississants, les tensio-actifs dont les tensio-actifs anioniques, cationiques, amphotères ou non ioniques, les humectants, les agents mouillants, les agents dispersants, les parfums, les pigments organiques ou encore minéraux comme les oxydes de fer, les agents huileux comme les huiles ou des graisses d'origine végétale, les graisses d'origine animale, les huiles de synthèse comme la vaseline, les huiles de silicone (cyclométhicone), les esters d'alcool gras, des huiles fluorées, des cires, des argiles modifiées, des bentones, des sels métalliques d'acide gras, la silice, les poly-éthylènes, du mica, les agents conservateurs, les agents antimicrobiens, des véhicules tels qu'une eau minérale, thermale ou florale, et/ou d'autres substances utilisées couramment en formulation dans le domaine cosmétique ou pharmaceutique.
L'extrait d'*Anigozanthos flavidus* peut être incorporé dans n'importe quel type de composition cosmétique. De préférence, il s'agit d'une composition cosmétique ayant une forme adaptée à l'administration topique, en particulier adaptée à une application sur la peau. Ladite composition cosmétique peut se présenter sous la forme de solutions aqueuses, hydroalcooliques, d'émulsions huile-dans-eau (H/E) ou eau-dans huile (E/H) ou multiple (triple : E/H/E ou H/E/H), des nanoémulsions, en particulier des nanoémulsions H/E, dont la taille des gouttes est inférieure à 100nm, de gels aqueux, de dispersions ou encore d'une poudre. La composition cosmétique selon l'invention peut se présenter sous la forme d'une lotion, d'un lait, d'une crème, d'un onguent, d'un gel, d'une mousse, d'une solution, d'une pommade. De manière plus générale, la composition selon l'invention peut se présenter également sous la forme d'un produit cosmétique de tout type. Il peut s'agir d'un soin cosmétique ou d'un produit de maquillage ou d'hygiène corporelle, par exemple une lotion, un lait, un sérum, un gel aqueux ou huileux, une émulsion, une crème, un gel-crème, une eau de soin, une pommade, un baume, un fond de teint, un spray, un ombre à paupière, un stick, un rouge à lèvre, un gloss, une mousse, un déodorant, un masque nourrissant, un gel douche, et un produit exfoliant ou gommant. A titre illustratif et non-limitatif, l'extrait d'*Anigozanthos flavidus* peut être présent à titre d'agent antiâge, reliftant ou remodelant dans une crème de jour destinée aux peaux matures ou âgées. L'extrait *d'Anigozanthos flavidus* peut être présent à titre d'agent antiâge dans un sérum régénérant destiné au visage ou au cou. A titre d'exemple supplémentaire, l'extrait d'*Anigozanthos flavidus* peut être présent en tant qu'actif lissant ou remodelant dans une crème de soin pour les mains, le cou ou le buste.

Dans certains modes de réalisation, l'extrait d'*Anigozanthos flavidus* est présent dans la composition cosmétique en association avec un agent de vectorisation. Dans certains modes de réalisation, l'extrait d'*Anigozanthos flavidus* est formulé, à l'aide d'un système de vectorisation. Le système de vectorisation peut être choisi parmi le groupe constitué par les micelles, les liposomes, y compris les liposomes unilamellaires ou multilamellaires, les niosomes, les éthosomes, les systèmes lamellaires, les nanosomes, les vésicules lipidiques ou polymériques, les nanosphères, les micro- ou nano-particules de polymères naturels ou non, les hydrogels. De préférence, l'extrait *d'Anigozanthos flavidus* est encapsulé dans un système de vectorisation choisi parmi les liposomes et les systèmes lamellaires. Des exemples particuliers de liposomes et de systèmes lamellaires pour la mise en œuvre de la présente invention sont décrits, entre autres, dans les demandes de brevet français n° 1358589 et n°1262303.

Un objet supplémentaire selon l'invention est un procédé cosmétique pour traiter ou prévenir un signe du vieillissement cutané chez un individu, comprenant l'administration d'une quantité cosmétiquement efficace de l'extrait d'*Anigozanthos flavidus,* de préférence par voie topique, audit individu. L'extrait d'*Anigozanthos flavidus* est typiquement appliqué sur la peau à traiter, par exemple au niveau du visage, du cou ou des mains. De préférence, l'extrait d'*Anigozanthos flavidus* est présent dans une composition cosmétique telle que décrite précédemment, et est donc appliqué sous la forme d'une composition cosmétique, par exemple sous la forme d'une crème. Comme mentionné ci-avant, le signe du vieillissement cutané peut résulter d'une altération des propriétés mécaniques de la peau, notamment une perte d'élasticité de la peau. De préférence, le procédé cosmétique selon l'invention a pour but de traiter ou prévenir un signe du vieillissement cutané choisi parmi l'apparition de ridules et/ou de rides, de tâches pigmentaires sur la peau, un flétrissement cutané, un affaissement ou un relâchement cutané, une perte de densité de la peau, une perte de fermeté de la peau, une perte de tonicité de la peau, une perte d'élasticité de la peau, un relâchement de la peau, une altération de l'aspect lisse de la peau, une augmentation de la rugosité de la peau, une altération du contour du visage et les combinaisons de ceux-ci. L'invention a également pour objet un procédé cosmétique pour exercer un effet tenseur immédiat sur la peau, de préférence sur une peau mature, comprenant l'administration d'une quantité cosmétiquement efficace de l'extrait d'*Anigozanthos flavidus,* de préférence par voie topique, audit individu. L'extrait d'*Anigozanthos flavidus* est typiquement appliqué sur la peau à traiter, par exemple au niveau du visage, du cou ou des mains.
Divulgué ici est également un procédé cosmétique pour réorganiser ou restructurer la matrice extracellulaire de la peau et/ou pour stimuler la synthèse d'une protéine choisie parmi le pro-collagène I, le collagène XVII, et la tenascine-X au niveau de la peau, de préférence au niveau d'une peau mature, et/ou pour augmenter les forces contractiles des fibroblastes dermiques d'une peau mature, ledit procédé comprenant l'administration d'une quantité cosmétiquement efficace de l'extrait d'*Anigozanthos flavidus,* de préférence par voie topique, audit individu. Dans un autre mode de réalisation, l'extrait d'*Anigozanthos flavidus* est utilisé pour stimuler le métabolisme du derme ou pour réorganiser ou restructurer le derme.
Dans les méthodes et utilisations cosmétiques selon l'invention, la dose à administrer et la fréquence d'administration de l'extrait *d'Anigozanthosflavidus* varient en fonction de l'effet cosmétique recherché, des caractéristiques de l'individu, en particulier de son sexe, de son âge et de son type de peau. Typiquement, l'extrait d'*Anigozanthos flavidus* peut être appliqué, sur la zone à traiter, une à deux fois par jour, typiquement le matin et/ou le soir, pendant plusieurs semaines consécutives voire plusieurs mois, par exemple au moins pendant 3 mois. La zone à traiter est typiquement choisie dans le groupe constitué par les mains, le cou, le visage ou une partie du visage telle que le contour des yeux, le contour des lèvres et la région des sillons nasogéniens. L'extrait d'*Anigozanthos flavidus* peut être appliqué sous la forme d'une composition cosmétique telle que décrite ci-après. Ladite composition cosmétique comprend typiquement de 0,001% à 10%, par exemple de 0,01% à 5% en poids d'extrait.
A titre d'exemple, pour obtenir un effet antiâge au niveau du visage, le patient peut appliquer une dose de 1 g à 2 g de composition cosmétique à 0,1% en poids d'extrait d'*Anigozanthos flavidus* sur son visage, matin et soir.
L'individu peut être une femme ou un homme, typiquement de plus de 30 ans, de préférence de plus de 40 ans ou de plus de 45 ans. Par exemple, l'individu peut être une femme de plus de 45 ans.

### - Objets supplémentaires selon l'invention

Selon un aspect supplémentaire, l'invention a également pour objet une composition cosmétique comprenant un extrait *d'Anigozanthos flavidus.* Typiquement, ladite composition cosmétique comprend :
- de 0,0001% à 10% d'extrait d'*Anigozanthos flavidus,*
- de 0% à 20% d'un ou plusieurs agents actifs additionnels, et
- de 70% à 99,9999% d'un ou plusieurs excipients acceptables sur le plan cosmétique,
   les pourcentages étant exprimés en poids par rapport au poids total de la composition cosmétique.
Dans certains modes de réalisation, ladite composition cosmétique comprend
- de 0,001% à 5% en poids d'extrait d'*Anigozanthos flavidus,*
- de 0,001% à 10% d'un ou plusieurs agents actifs additionnels, et
- de 85% à 99,998 % d'un ou plusieurs excipients acceptables sur le plan cosmétique.
Dans d'autres modes de réalisation, ladite composition cosmétique comprend :
- de 0,01% à 5% en poids d'extrait d'*Anigozanthos flavidus,*
- de 0,01% à 10% d'un ou plusieurs agents actifs additionnels, et
- de 85% à 99,98 % d'un ou plusieurs excipients acceptables sur le plan cosmétique.

Il va de soi que l'extrait, les agents actifs additionnels et les excipients acceptables sur le plan pharmaceutique sont tels que décrits ci-avant. De même, la composition cosmétique peut être de tout type, comme expliqué précédemment.
En particulier, l'extrait d'*Anigozanthos flavidus* peut être sous la forme d'un extrait liquide. Dans un mode de réalisation complémentaire ou alternatif, la composition cosmétique comprend de 0,001% à 10%, par exemple de 0,005% à 1% en poids de matière sèche issue de l'extrait *d'Anigozanthos flavidus.*

Divulguée ici est également une composition destinée à être incorporée dans une composition cosmétique. Cette composition correspond donc à une composition « *précurseur ».*
De préférence, cette composition pour la préparation d'une composition cosmétique comprend :
- de 10% à 50%, de préférence de 20% à 40% d'un extrait d'*Anigozanthos flavidus,*
- de 50% à 90%, de préférence de 60% à 80% d'un véhicule, de préférence choisi parmi un solvant aqueux, un solvant organique, de préférence un alcool inférieur tel que l'éthanol, le propanediol, le butylène glycol, la glycérine ou l'isopropanol, un agent lipophile et leurs mélanges, et
- en option, de 0,01 à 30% d'un excipient supplémentaire acceptable sur le plan cosmétique, de préférence choisi parmi un ajusteur de pH, un agent tampon un agent de vectorisation, un agent antioxydant, un agent de vectorisation, un agent conservateur, un agent stabilisant, un agent épaississant, un émulsifiant, un agent gélifiant hydrophile ou lipophile, un parfum, une huile minérale ou organique, et leurs combinaisons.
les pourcentages étant exprimés en poids par rapport au poids total de la composition.
Il va de soi que l'extrait *d'Anigozanthos flavidus* peut être tel que décrit précédemment. A titre d'exemple, l'extrait d'*Anigozanthos flavidus* peut être un extrait susceptible d'être obtenu à partir d'une partie aérienne d'*Anigozanthos flavidus* par extraction avec un solvant polaire. Dans certains modes de réalisation, l'extrait d'*Anigozanthos flavidus* est un extrait liquide, par exemple dans l'eau ou dans un mélange hydro-alcoolique tel qu'un mélange eau/glycérol.
Dans un mode de réalisation particulier, la composition pour la préparation d'une composition cosmétique destinée au traitement ou à la prévention d'un signe du vieillissement comprend:
- de 20% à 40 % en poids d'un extrait liquide obtenu à partir d'une partie aérienne d'*Anigozanthos flavidus* en tant qu'agent antiâge, et
- de 60% à 80% en poids de glycérol,
les pourcentages étant exprimés en poids par rapport au poids total de la composition.
La composition « précurseur » peut, en outre comprendre, un agent tampon ou un agent ajusteur de pH tel que l'acide citrique. Typiquement, la composition « précurseur » selon l'invention a un pH compris entre 3,0 et 4,5, typiquement d'environ 3,9.
Dans un mode de réalisation complémentaire ou alternatif, la composition « précurseur » comprend de 0,01% à 10%, par exemple de 0,05% à 5% en poids ou encore de 0,1% à 1% en poids de matière sèche issue de l'extrait d'*Anigozanthos flavidus.*
La présente invention a également pour objet l'utilisation de ladite composition « précurseur » pour la préparation d'une composition cosmétique. Il va de soi que ladite composition cosmétique finale est particulièrement adaptée pour la mise en œuvre de l'une quelconque des utilisations cosmétiques selon l'invention, et peut présenter l'une quelconque des caractéristiques décrites ci-avant.
La composition « précurseur » peut être présente dans la composition cosmétique à hauteur de 0,001% à 50%, de préférence de 0,01% à 10% en poids, par exemple de 0,5% à 5% ou encore de 1,5% à 2,5% en poids par rapport au poids total de la composition cosmétique ou pharmaceutique finale.
Ladite composition cosmétique peut être obtenue en mélangeant une composition « précurseur » selon l'invention avec un ou plusieurs excipients sur le plan cosmétique et/ou avec un ou plusieurs actifs additionnels à effet cosmétique.

Divulguée ici est également une méthode pour préparer une composition cosmétique selon l'invention comprenant une étape dans laquelle un extrait d'*Anigozanthos flavidus* ou la composition « précurseur » est mélangé(e) avec un ou plusieurs excipients acceptables sur le plan cosmétique et/ou avec un ou plusieurs actifs à effet cosmétique additionnels.
Divulguée ici est également une méthode de préparation d'une composition « précurseur » selon l'invention comprenant :
a) La fourniture d'une matrice végétale d'*Anigozanthos flavidus* de préférence issue d'une partie aérienne d'*Anigozanthos flavidus* par séchage et broyage
b) L'extraction de la matrice végétale par un solvant polaire suivie d'une étape de filtration de manière à obtenir un extrait liquide,
c) L'addition d'un véhicule acceptable sur le plan cosmétique dans l'extrait liquide issu de la filtration,
d) En option, l'addition d'un excipient acceptable sur le plan cosmétique dans le filtrat,
e) En option, une étape d'ajustement du pH,
Les étapes c), d) et e) pouvant être réalisées dans n'importe quel ordre.
Dans certains modes de réalisation, le véhicule acceptable sur le plan cosmétique comprend de la glycérine et/ou de l'eau.
L'étape d'extraction peut être effectuée comme décrit ci-avant, par exemple en utilisant l'eau ou un mélange hydroalcoolique en tant que solvant polaire.
D'autres aspects et avantages de la présente invention apparaîtront à la lecture des exemples qui suivent, qui doivent être considérés comme illustratifs et en aucun cas comme limitatifs.

### EXEMPLES

Les Exemples qui suivent ont été réalisés avec une composition nommée ci-après « Extrait », ladite composition « Extrait » comprenant :
- 30 % en poids d'un extrait aqueux de fleurs et de tiges *d'Anigozanthos flavidus* à environ 1,6 % en poids de matière sèche, et
- de 70% en poids de glycérine.
Il s'agit d'une composition « précurseur » au sens de l'invention.

### EXEMPLE 1 : Effet d'un extrait d'Anigozanthos flavidus sur la synthèse du pro-collagène I par des fibroblastes dermiques humains

### - Contexte

Les collagènes constituent une famille de protéines fortement impliquées dans les propriétés mécaniques de la peau. Le pro-collagène I est le précurseur du collagène de type I, qui est un constituant majeur du tissu conjonctif. Avec l'âge, la teneur en collagène dans la peau diminue, ce qui conduit à la formation de rides ou de ridules au niveau de la peau.
Le but de cette étude était de déterminer les effets d'un extrait d'*Anigozanthos flavidus* sur la synthèse de pro-collagène par des fibroblastes du derme humain.

### - Protocole

La composition « Extrait » a été testée aux concentrations suivantes 0,1% et 0,05% (v/v). Le TGF-β a été choisi en tant que témoin positif et testé à 10 ng/ml. Le témoin négatif correspond à des cellules non-traitées par la composition « Extrait » ou le TGFβ.
Les fibroblastes ont été isolés à partir de derme humain et maintenus dans un milieu de culture spécifique DMEM supplémenté avec 10% de sérum de veau fœtal, 1% d'antibiotiques (pénicilline/ streptomycine) et 1% de L-glutamine, à 37 ° C, sous 5% de CO₂ et 95% d'humidité.
2,5 x 10⁴ cellules ont été ensemencées par puit de microplaque, dans un milieu DMEM complet. Les cellules ont été mises en incubateur pendant 24h. Le milieu de culture a été ensuite remplacé par du DMEM sans sérum. Les microplaques ont été laissées 24 heures supplémentaires en incubateur pour atteindre la quiescence cellulaire.
La composition « Extrait » ou le TGF-β ont été ajoutés à la concentration souhaitée. Les microplaques ont été mises en incubation pendant 24 heures supplémentaires. A l'issue de l'incubation, les surnageants ont été prélevés et analysés par ELISA (MK101Z, OZYME) afin de quantifier le pro-collagène I.

### - Résultat

Les résultats obtenus sont illustrés à la Figure 1. La Figure 1 montre le pourcentage d'activation de la synthèse de pro-collagen I dans les cellules traitées par rapport aux cellules non-traitées.
Les fibroblastes traités avec la composition « Extrait » ou le TGFβ ont produit une quantité plus importante de pro-collagène I que les cellules non-traitées. L'effet de la composition « Extrait » sur la synthèse de collagène I est dose-dépendante. L'extrait d'*Anigozanthus flavidus* stimule donc la synthèse de pro-collagène I chez les fibroblastes du derme.

### EXEMPLE 2 : Effet d'un extrait d'Anigozanthos flavidus sur la synthèse de Tenascine X par des fibroblastes dermiques humains

La Ténascine-X est une protéine qui régule à la fois la structure et la stabilité des fibres élastiques de la matrice extracellulaire (ECM). Elle participe également à l'organisation des fibres de collagène. La tenascine-X joue un rôle clé dans la rigidité et l'élasticité des tissus conjonctifs. Une déficience hériditaire en tenascine-X peut conduire à une maladie génétique appelée syndrome d'Ehlers-Danlos.
Le but de cette étude était de déterminer l'effet de la composition « Extrait » sur la synthèse de la ténascine-X par les fibroblastes du derme humain.

### - Protocole

La composition « Extrait » a été testée à 0,2% et 0,1% (v/v). Le sérum fœtal de veau à 10% a été utilisé en tant que témoin positif. Le témoin négatif correspond à des cellules non-traitées par l'Extrait ou par le sérum de veau fœtal.
Les fibroblastes ont été isolés à partir de derme humain abdominal et maintenus dans un milieu de culture spécifique DMEM supplémenté avec 10% de sérum de veau fœtal, 1% d'antibiotiques (pénicilline/ streptomycine) et 1% de L-glutamine, à 37 ° C, sous 5% de CO₂ et 95% d'humidité.
2,5 x 10⁴ cellules ont été ensemencées par puit de microplaque, dans un milieu DMEM complet. Les cellules ont été mises en incubateur pendant 24h. Le milieu de culture a été ensuite remplacé par du DMEM sans sérum. Les microplaques ont été laissées 24 heures supplémentaires en incubateur pour atteindre la quiescence cellulaire.
La composition « Extrait » ou le sérum de veau fœtal ont été ajoutés à la concentration souhaitée. Après l'ajout, les cellules ont été incubées pendant 24h supplémentaires. A l'issue de l'incubation, les surnageants ont été prélevés et analysés par ELISA afin de quantifier la tenascine-X. Parallèlement, à l'issue de l'incubation, les protéines contenues dans les lysats cellulaires ont été quantifiées par la méthode de Bradford.

### - Résultats

Les résultats obtenus ont été soumis à un test de Student-t pour une p-valeur < 0,05. Les résultats sont présentés dans la Figure 2. La Figure 2 montre pour chaque condition expérimentale la quantité de tenascine-X présente dans le milieu, exprimée en pourcentage par rapport à la quantité totale de protéines. On note que la composition « Extrait » induit une augmentation dose-dépendante de la quantité de tenascine-X, par rapport au témoin négatif (cellule non-traitée). En d'autres termes, l'extrait d'*Anigozanthos flavidus* stimule la synthèse de tenascine-X dans les fibroblastes humains.

### EXEMPLE 3 : Effet d'un extrait d'Anigozanthos flavidus sur la synthèse de collagène XVII les kératinocytes humains

Le collagène XVII est une protéine transmembranaire qui joue un rôle essentiel dans le maintien de la liaison entre des éléments intracellulaires et extracellulaires impliqués dans l'adhésion de l'épiderme.
Le but de cette étude était de déterminer l'effet de la composition « Extrait » sur la synthèse du collagène XVII par les kératinocytes humains.

### - Protocole

La composition « Extrait » a été testée à une concentration de 0,4% (v/v). Le TGF-β a été choisi en tant que témoin positif et testé à 10 ng/ml. Le témoin négatif correspond à des cellules non-traitées par l'Extrait ou le TGF-β.
Les kératinocytes ont été isolés à partir d'une lignée cellulaire épithéliale humaine et mis en culture dans du DMEM supplémenté avec 10% de sérum de veau fœtal, 1% d'antibiotiques (pénicilline/ streptomycine) et 1% de L-glutamine, à 37 °C, sous 5% de CO₂ et 95% d'humidité.
5 x 10⁴ cellules ont été ensemencées par puit de microplaque, dans du milieu DMEM complet et laissées en incubateur pendant 24h. Le milieu de culture a été ensuite remplacé par du DMEM comprenant 1% de sérum de veau fœtal, 1% d'antibiotique et 1% de L-glutamine. Les microplaques ont été laissées 24 heures supplémentaires en incubateur.

A l'issue de l'incubation, la composition « Extrait » ou le TGF-β ont été ajoutés à la concentration souhaitée. Les microplaques ont été mises en incubation pendant 24 heures supplémentaires.
Le collagène XVII a été détecté et quantifié par immuno-marquage à l'aide d'un anticorps anti-collagène XVII dilué au 1/100 et révélé par un anticorps secondaire couplé à la fluorescéine. L'intensité de fluorescence correspond au niveau d'expression du collagène XVII. Les images ont été traitées par le logiciel Image J.

### - Résultats

Les mesures de fluorescence ont été normalisées par rapport à la mesure de l'intensité de la fluorescence mesurée pour le contrôle négatif. On observe une augmentation de +27% l'intensité de la fluorescence pour les cellules incubées avec la composition « Extrait », ce qui illustre une augmentation de l'expression de collagène XVII par rapport aux cellules non-traitées. L'incubation avec le TGF-β (contrôle positif) a entraîné une augmentation de 257% du signal de fluorescence par rapport au témoin négatif.
En résumé, l'extrait *d'Anigozanthos flavidus* stimule la synthèse du collagène XVII par les kératinocytes humains.

### EXEMPLE 4 : Evaluation de l'effet tenseur de l'Extrait : quantification des forces de contraction générées par des fibroblastes issus de fond de ride et quantification de l'a-SM actine

Grâce au système GlasBox^{PlusTM}, il est possible de quantifier l'effet tenseur d'un actif cosmétique sur un derme « équivalent » obtenu à partir de fibroblastes issus de fond de ride. Les fibroblastes issus de fond de ride développent moins de forces contractiles que ceux de la peau non ridée avoisinante. Il est également possible de réaliser des immuno-marquage de l'a-SM actine au sein des dermes équivalents après les mesures des forces contractiles dans le système GlasBox^{PlusTM}. Cette étude avait pour but de montrer l'effet de l'Extrait sur la contractilité des fibroblastes et sur la synthèse de l'a-SM actine, qui est impliquée dans la plasticité, la migration et la motilité cellulaire.

### ▪ Protocole

### - Culture des fibroblastes

Des biopsies de 2 mm de diamètre sont réalisées sur un lifting (déchet opératoire ; femme de 63 ans) au niveau de la peau non ridée et au sein d'une ride.

La technique d'explantation classique est utilisée pour l'extraction des fibroblastes de la peau saine (FS) et issus de fond de ride (FR). Les explants sont mis en culture dans le milieu de "Dulbecco's modified Eagle's medium" complémenté avec 10% de sérum de veau foetal (SVF), 40 mg/l de gentamicine et 2 mg/l de fungizone (DMEMc) à 37°C, en atmosphère humide et en présence de 5% de CO2. Progressivement (en une dizaine de jours), les fibroblastes migrent à l'extérieur des explants. Le milieu de culture est renouvelé deux fois par semaine. Lorsque les fibroblastes sont en quantité suffisante, ils sont décollés sous l'action de la trypsine-EDTA, repiqués et amplifiés dans les mêmes conditions de culture.

### - Système GlasBoxPlus™ et mesure des forces contractiles

Les dermes équivalents se développent dans une boîte de culture qui est constituée de huit cuves rectangulaires. Dans chacune d'elles plongent deux lames flexibles dont les parties inférieures sont constituées de grilles sur lesquelles s'accroche le derme équivalent lors de sa polymérisation. Le derme équivalent se développe entre deux lames pour aboutir à une forme rectangulaire légèrement rétrécie au centre. Cette forme en mécanique classique est désignée comme une forme en "diabolo".
Sous l'influence des forces de contraction développées par les fibroblastes, les lames se déforment. Leur déformation est mesurée à l'aide de fibres optiques. Cette déformation est proportionnelle à la force développée au sein du derme équivalent. Les mesures sont effectuées en temps réel à l'aide d'une carte d'acquisition PC et d'un logiciel adapté.
A confluence, les fibroblastes cultivés en monocouche sont trypsinés puis comptés à l'aide d'une lame de Mallassez pour amener la suspension cellulaire à 8.10⁵ cellules/ml. Un milieu de fabrication des dermes équivalents est préparé en mélangeant 6 volumes de milieu 1,76X (DMEMc, NaHCO3, NaOH, antibiotiques, SVF), 3 volumes de collagène de type I de queue de rat (2 mg/ml) et 1 volume de suspension cellulaire (8.105 cellules/ml)
Le mélange est coulé dans les cuves rectangulaires de la GlasBox^{PlusTM}. En quelques minutes à 37°C, un gel se constitue. Les différents milieux de culture contenant la composition « Extrait » (concentration finale dans le milieu : 0,1% et 0,2% (V/V)), le TGF β1 (2,5 ng/ml) (témoin positif) ou dépourvu (contrôle négatif) sont ajoutés et les forces isométriques sont mesurées pendant 24 heures.

### - Marquage de l'α-SM Actine

L'immunomarquage des fibres d'a-SM actine est réalisé grâce à l'utilisation d'un anticorps monoclonal de souris anti-α-SM actine. Un deuxième anticorps anti-souris couplé à la FITC permet la réalisation et la localisation de l'immunomarquage. Les noyaux ont été marqués au colorant Hoechst. L'observation a été réalisée en microscopie confocale à 488 nm et biphotonique à 705 nm d'excitation.
Des fragments de dermes équivalents sont obtenus à l'aide d'un punch biopsy de 5 mm de diamètre. Les dermes équivalents sont placés dans l'acétone à froid afin de les perméabiliser. Après lavage au PBS, une solution d'eau oxygénée est ajoutée afin d'éliminer les peroxydases endogènes. Après rinçage au PBS, les dermes équivalents sont placés dans une solution de glycine pour éliminer les bruits de fond dû aux groupements aldéhyde du fixateur. Les dermes équivalents sont ensuite placés dans une solution de blocage permettant de bloquer les liaisons aspécifiques qui seraient dues à l'anticorps secondaire. Les tissus sont placés pendant une nuit dans l'anticorps primaire. Après lavage au PBS, l'anticorps secondaire est ajouté. Après une heure d'incubation et lavage au PBS, les noyaux sont marqués au colorant Hoechst pendant 10 min.
Après rinçage dans le PBS, les dermes équivalents sont montés entre lame et lamelle à l'aide d'Eukitt. Les lames sont observées au microscope confocale (Zeiss LSM510 NLO) au grossissement x40 et des photographies sont prises.

### - Analyses statistiques

Pour l'étude des forces contractiles, une analyse de variance à deux facteurs est réalisée, suivie si nécessaire d'un test de Fisher. Les significativités ne sont retenues que lorsque p<0,05. Les aires sous la courbe (AUC) sont calculées ainsi que la pente (vitesse de contraction) et le maximum de contraction, le tout à l'aide du logiciel GraphPad Prism® 5. Pour ces données, une analyse de variance à un facteur est réalisée, suivie si nécessaire d'un test de Fisher. Les significativités ne sont retenues que lorsque p<0,05.

### Résultats

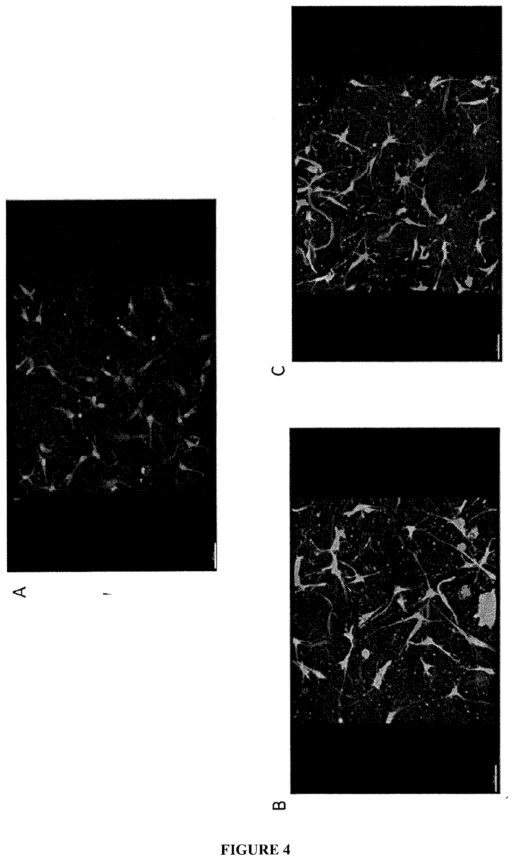

Les forces contractiles développées par les fibroblastes de fond de ride (FR) sont significativement moins importantes que celles développées par les fibroblastes sains (FS). On observe que les AUC (aire sous courbe de la courbe représentant la force contractile en fonction du temps) ainsi que le maximum de contraction sont significativement diminués pour les fibroblastes FR par rapport aux fibroblastes FS (données non-montrées). L'incubation des fibroblastes avec le TGFβ1 induit une augmentation significative de l'AUC et de la force contractile maximale aussi bien chez les fibroblastes FR que chez les fibroblastes FS.
L'incubation avec la composition « Extrait » entraîne une augmentation significative des forces contractiles dans le groupe des fibroblastes FR : la force contractile maximale et l'AUC sont significativement augmentées. Ces résultats sont illustrés par les Figures 3A, 3B et 3C. La composition « Extrait » augmente également de manière globale les forces contractiles dans les fibroblastes FS, mais l'augmentation observée est plus faible que celle obtenue pour les fibroblastes FR. Les expériences d'immuno-marquage montrent que la composition « Extrait » stimule l'expression de la α-SM actine par les fibroblastes FR (cf Figure 4).

L'extrait d'*Anigozanthos flavidus* stimule significativement et spécifiquement les forces contractiles des fibroblastes issus de peau ridée. Il exerce donc un effet tenseur, liftant, sur les peaux matures ou âgées.

### EXEMPLE 5 : Evaluation de l'effet tenseur physique, immédiat, de l'Extrait.

Grâce au système GlasBox^{Plus™}, il a également été possible de quantifier l'effet tenseur immédiat de l'Extraitsur un derme équivalent acellulaire, en mesurant les forces contractiles générées.

### ▪ Protocole

### - Système GlasBoxPlus™ et mesure des forces contractiles

Les dermes équivalents acellulaires polymérisent dans une boite de culture qui est constituée de huit cuves rectangulaires. Dans chacune d'elles plongent deux lames flexibles dont les parties inférieures sont constituées de grilles sur lesquelles s'accroche le derme équivalent acellulaire lors de sa polymérisation. La polymérisation du gel de collagène en présence ou non de l'Extrait engendre des forces qui provoquent la déformation des lames. Cette déformation est mesurée à l'aide de fibres optiques. Elle est proportionnelle à la force développée au sein du derme équivalent acellulaire.

Un milieu de fabrication des dermes équivalents a été préparé en mélangeant 6 volumes de milieu 1,76X (DMEMc, NaHCO3, NaOH, antibiotiques, SVF), de 3 volumes de collagène de type I de queue de rat (2 mg/ml) et d'1 volume de DMEMc.
Le mélange a été coulé dans les cuves rectangulaires de la GlasBox^{PlusTM}. En quelques minutes à 37°C, un gel se constitue. Les différents milieux de culture contenant la composition « Extrait » (concentration finale dans le milieu : 0,2% (V/V)) ou dépourvu (contrôle négatif) ont été ajoutés et les forces isométriques ont été mesurées pendant 24 heures.

### - Analyses statistiques

Pour l'étude des forces contractiles, une analyse de variance à deux facteurs a été réalisée, suivie si nécessaire d'un test de Fisher p<0,05.
Les aires sous la courbe (AUC), la pente (vitesse de contraction) et le maximum de contraction ont été calculées à l'aide du logiciel GraphPad Prism® 5. Pour ces données, une analyse de variance à un facteur a été réalisée, suivie si nécessaire d'un test de Fisher (p<0,05).

### Résultats

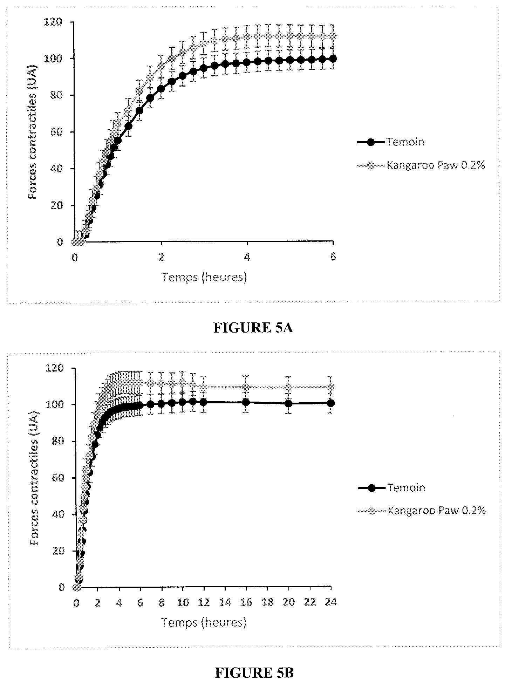

Les forces contractiles sont significativement augmentées en présence de l' « Extrait » à 0,2% par rapport au groupe Témoin. Cette augmentation est significative 1h30 après le début de la mesure et se prolonge au moins jusqu'à la 11^{ème} heure en présence de 0.2% d' Extrait (Figures 5A et 5B)). On observe également que l'AUC (aire sous la courbe représentant la force contractile en fonction du temps) ainsi que le maximum de contraction sont significativement augmentés pour le groupe traité avec l'Extrait selon l'invention par rapport au groupe témoin, non traité avec l'Extrait.

L'Extrait d'*Anigozanthos flavidus* stimule significativement et spécifiquement les forces contractiles des dermes équivalents acellulaires. L'Extrait d'*Anigozanthos flavidus* selon l'invention exerce donc un effet physique astringent, tenseur, et liftant immédiat et peut donc être utilisé pour exercer un effet tenseur immédiat sur les peaux matures.

### EXEMPLE 6 : Exemples de compositions cosmétiques comprenant l'Extrait

### - Crème antiâge (Crème B) comprenant 2% de la composition « Extrait »

| Phase | Nom INCI | % en poids |
|---|---|---|
| A | Eau | 89,40% |
| | EDTA tétrasodique | 0,10% |
| | Chlorophénésine | 0,30% |
| | Phénoxyéthanol | 0,80% |
| B | Copolymer d'acrylates de sodium et lécithine (Lecigel®) | 2,00% |
| C | Dicaprylyl carbonate | 3,00% |
| | Huile d'amande douce | 2,00% |
| | tocophérol et extrait de tournesol (Vitapherole® E1000 | 0,20% |
| D | Parfum | 0,20% |
| | Glycérine et extrait d'*Anigozanthos flavidus* (composition « Extrait ») | 2,00% |

Les propriétés antiâges de la crème B ont été évaluées dans l'essai clinique présenté dans l'Exemple 7.

### - Serum antiâge comprenant 2% de la composition « Extrait »

| Phase | Nom INCI | % en poids |
|---|---|---|
| A | Eau | 83,67% |
| | Phytate de Sodium et eau et alcool (Dermofeel® PA-3) | 0,15% |
| | Eau et Cl 14700 | 0,28% |
| | Lysolecithine et gomme de Sclerotium et Gomme de Wanthan et Pullulane (Ecogel™) | 2,00% |
| B | Isoamyl Laurate (Dermofeel® Sensolv) | 1,00% |
| | Beurre de Karité (Lipex® 102) | 3,00% |
| | Huile d'amande douce | 3,00% |
| | Tocophérol et extrait de tournesol (Vitapherole® E1000 | 0,20% |
| C | Glycerine et eau et lévulinate de sodium et Anisate de sodium | 4,00% |
| | Glycérine et extrait d'*Anigozanthos flavidus* (composition « Extrait ») | 2,50% |
| D | Parfum | 0,20% |

### - Un baume antiâge comprenant 2% de la composition « Extrait »

| Phase | Nom INCI | % en poids |
|---|---|---|
| A | Eau | 76,90% |
| | Phytate de Sodium et eau et alcool (Dermofeel® PA-3) | 0,10% |
| | Glycérine | 5,00% |
| | Chlorphenesion | 0,30% |
| | Phénoxyéthanol | 0,80% |
| B | Copolymer d'acrylates de sodium et lécithine (Lecigel®) | 2,00 % |
| C | Beurre de Karité (Lipex® 102) | 5,00% |
| | Alcool béhénylique | 2,00% |
| | Isononanoate d'isononyle Glyceryl Stearate citrate et Polyglyceryl-3 | 5,00% |
| | sterate et lecithin hydrogénée (Heliofeel ™) | 1,00% |
| | Tocophérol et extrait de tournesol (Vitapherole® E1000 | 0,20% |
| D | Glycérine et extrait d'*Anigozanthos flavidus* (composition « Extrait ») | 1,50% |
| | Parfum | 0,20% |

### EXEMPLE 7 : Evaluation clinique de l'effet antiride de l'Extrait.

### Crèmes testées :

- Crème B (voir Exemple 5) comprenant 2% de la composition « Extrait »
- Crème A : Crème Placebo ayant la même composition que la crème B à part que les 2% de la composition « Extrait » ont été remplacés par 2% d'eau déionisée.

### Protocole :

Un test d'usage du produit **Crème A** (crème placébo) a été réalisé en comparaison du produit **Crème** B contenant 2% de la composition « Extrait », selon le protocole suivant:
42 volontaires en aveugle ont été incluses dans l'étude et réparties en deux groupes selon une liste de randomisation établie par un programme dédié:
- Groupe A recevant la crème **A** : 21 femmes âgées de 45 à 69 ans (moyenne d'âge : 56,4 ans), présentant tous types de peaux, dont 57% de peaux sensibles, de phototypes II à IV.
- Groupe B recevant la crème **B** : 21 femmes âgées de 46 à 77 ans (moyenne d'âge : 60,8 ans), présentant tous types de peaux, dont 48% de peaux sensibles, de phototypes II à IV.
Le produit a été appliqué la première fois par les volontaires au laboratoire. Les applications suivantes ont été réalisées chez les volontaires, par auto-application, à raison de deux applications par jour. T+1h après la première application : Evaluation de la tolérance du produit à l'essai par appréciation des paramètres cutanés par le dermatologue sur la zone d'intérêt après retrait du produit. T+7 jours : Evaluation de la tolérance du produit à l'essai par appréciation des paramètres cutanés sur la zone d'intérêt après 7 jours d'utilisation (par contact téléphonique). T+28 jours: Evaluation de la tolérance du produit à l'essai par le dermatologue suivant l'appréciation des paramètres cutanés après 28 jours de test. Auto-évaluation par les volontaires des qualités du produit à l'essai à l'aide d'un questionnaire dédié.
Par ailleurs, une évaluation de l'efficacité du produit vis-à-vis de la profondeur moyenne des rides de la patte d'oie a été réalisée.
Les mesures d'efficacité ont été réalisées à T0, T1h (soit 1 heure après l'application) et T28 (soit après 28 jours d'application du produit testé). La mesure à 1h a permis d'évaluer l'effet tenseur immédiat de l'Extrait. La mesure à T28 a permis d'évaluer l'effet antiâge, notamment antiride, sur le long terme de l'Extrait. L'évaluation de la profondeur des rides a été réalisée en quantifiant le relief de la surface de la peau par le système LIFEVIZ Micro™ (QUANTIFICARE). LIFEVIZ Micro™ est une méthode de mesure sans contact basée sur la projection de lumière structurée sur les zones de la peau d'intérêt.

### Résultats

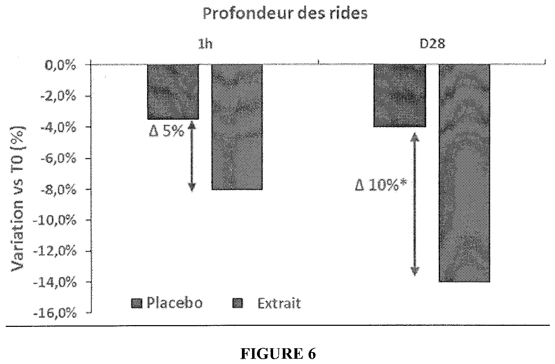

La crème B a été bien tolérée au cours de l'essai par les patientes.
Les peaux traitées par la **crème B** contenant l'extrait *d'Anigozanthos flavidus* ont montré une diminution de la profondeur des rides de 10% de façon significative après 28 jours d'application en comparaison avec les peaux traitées par la **crème A** (crème placébo) (Figure 6). Une diminution de la profondeur des rides de 5% est visible 1 heure après l'application de la crème B, soulignant l'effet tenseur immédiat de l'Extrait selon l'invention (Figure 6).

## Revendications

1. Utilisation cosmétique d'un extrait obtenu à partir d'une partie aérienne d'*Anigozanthos flavidus* en tant qu'agent antiâge.

2. Utilisation cosmétique d'un extrait *d'Anigozanthos flavidus* selon la revendication 1, en tant qu'agent anti-ride, lissant, tonifiant, défroissant, liftant ou reliftant, restructurant ou remodelant de la peau.

3. Utilisation cosmétique selon l'une quelconque des revendications précédentes dans laquelle l'extrait d'*Anigozanthos flavidus* est présent en tant qu'agent actif dans une composition cosmétique destinée à prévenir, retarder et/ou traiter un ou plusieurs signes du vieillissement cutané.

4. Utilisation cosmétique selon la revendication 3, dans laquelle le ou les signes du vieillissement cutané sont choisis parmi le groupe constitué par : l'apparition de ridules et/ou de rides sur la peau, notamment au niveau des lèvres et des paupières, un flétrissement ou un affaissement de la peau, notamment au niveau du cou, une perte d'éclat de la peau, un teint brouillé, les tâches pigmentaires, les cernes au niveau des yeux, une perte de densité de la peau, une perte de fermeté de la peau, une perte de tonicité de la peau, une perte d'élasticité de la peau, une perte de contractilité de la peau, une perte de maintien de la peau, un relâchement de la peau, une altération de l'aspect lisse de la peau, une augmentation de la rugosité de la peau, et une altération du contour du visage.

5. Utilisation cosmétique selon l'une quelconque des revendications 1 à 4, dans laquelle l'extrait d'*Anigozanthos flavidus* est utilisé pour améliorer, restaurer ou prévenir l'altération des propriétés mécaniques de la peau chez une peau mature.

6. Utilisation cosmétique selon l'une quelconque des revendications 1 à 4, dans laquelle l'extrait d'*Anigozanthos flavidus* est utilisé pour améliorer le teint ou l'éclat d'une peau mature.

7. Utilisation cosmétique selon l'une quelconque des revendications précédentes, dans laquelle l'extrait est obtenu à partir d'une partie aérienne *d'Anigozanthus flavidus* choisi parmi les fleurs, les feuilles, les tiges et leurs combinaisons.

8. Utilisation cosmétique selon l'une quelconque des revendications précédentes, dans laquelle l'extrait d'*Anigozanthos flavidus* est obtenu par extraction avec un solvant polaire.

9. Utilisation cosmétique selon l'une quelconque des revendications 3-8, dans laquelle la composition cosmétique comprend en outre, au moins un agent cosmétique additionnel, de préférence choisi dans le groupe constitué par les vitamines, les filtres et écrans solaires, les agents anti-âges, ou antirides, les antioxydants, les agents liftants, les agents raffermissants, les agents anti-tâches, les agents anti-rougeurs, les agents amincissants, les agents drainants, les agents hydratants, les agents apaisants, les agents gommants ou exfoliants, les agents matifiants, les agents séborégulateurs, les actifs éclaircissants, les actifs auto-bronzants, les accélérateurs de bronzage et leurs combinaisons.

10. Utilisation cosmétique selon l'une quelconque des revendications 3-9, dans laquelle la composition cosmétique est choisie parmi le groupe constitué par les solutions aqueuses, les solutions hydroalcooliques, les émulsions huile-dans-eau (H/E) ou eau-dans huile (E/H) ou multiple (triple : E/H/E ou H/E/H), les nanoémulsions, en particulier des nanoémulsions H/E, dont la taille des gouttes est inférieure à 100nm, les gels aqueux, ou les dispersions d'une phase grasse dans une phase aqueuse à l'aide de sphérules, les suspensions, de préférence en milieux aqueux ou hydroalcoolique, les suspensions de liposomes, les poudres, les lotions, laits, les crèmes, les onguents, les gels, les mousses, et les pommades.

11. Utilisation cosmétique selon l'une quelconque des revendications 3-10 dans laquelle la composition cosmétique est choisie parmi un produit cosmétique, un produit de maquillage ou un produit d'hygiène corporelle, par exemple une lotion, un lait, un sérum, un gel aqueux ou huileux, une émulsion, une crème notamment une crème de jour ou une crème de nuit, un gel-crème, une eau de soin, une pommade, un baume, un fond de teint, un spray, un ombre à paupière, un stick, un rouge à lèvre, un gloss, un baume à lèvres, une mousse, un déodorant, un masque nourrissant, un gel douche, et un produit exfoliant ou gommant.

12. Composition pour la préparation d'une composition cosmétique destinée au traitement ou à la prévention d'un signe du vieillissement comprenant :
- de 20% à 40% en poids d'un extrait liquide obtenu à partir d'une partie aérienne d'*Anigozanthos flavidus* en tant qu'agent antiâge, et
- de 60% à 80% en poids de glycérol.

## Patentansprüche

1. Kosmetische Verwendung eines Extrakts, erhalten aus einem oberirdischen Teil von *Anigozanthosflavidus,* als Anti-Aging-Mittel.

2. Kosmetische Verwendung eines Extrakts von *Anigozanthos flavidus* gemäß Anspruch 1, als Antifalten-, Glättungs-, Tonisierungs-, Entknitterungs-, Lifting- oder Relifting-, Restrukturierungs- oder Remodellierungsmittel der Haut.

3. Kosmetische Verwendung gemäß einem der vorhergehenden Ansprüche, wobei der Extrakt von *Anigozanthos flavidus* als Wirkstoff in einer kosmetischen Zusammensetzung zur Prävention, Verzögerung und/oder Behandlung eines oder mehrerer Anzeichen der Hautalterung vorliegt.

4. Kosmetische Verwendung gemäß Anspruch 3, wobei das oder die Anzeichen der Hautalterung aus der Gruppe ausgewählt sind, bestehend aus: Auftreten von Fältchen und/oder Falten auf der Haut, insbesondere um Lippen und Augenlider, einem Welken oder Erschlaffen der Haut, insbesondere am Hals, einem Verlust an Strahlkraft der Haut, einem unreinen Teint, Pigmentflecken, Augenringen, einem Verlust an Hautdichte, einem Verlust an Hautfestigkeit, einem Verlust an Spannkraft der Haut, einem Verlust an Elastizität der Haut, einem Verlust an Kontraktilität der Haut, einem Verlust an Erhaltung der Haut, einer Erschlaffung der Haut, einer Veränderung des glatten Aussehens der Haut, einer Zunahme von Hautunebenheiten und einer Veränderung der Gesichtskontur.

5. Kosmetische Verwendung gemäß einem der Ansprüche 1 bis 4, wobei der Extrakt von *Anigozanthosflavidus* zur Verbesserung, Wiederherstellung oder Prävention der Veränderung der mechanischen Eigenschaften der Haut bei einer reifen Haut verwendet wird.

6. Kosmetische Verwendung gemäß einem der Ansprüche 1 bis 4, wobei der Extrakt von *Anigozanthos flavidus* zur Verbesserung des Teints oder der Strahlkraft einer reifen Haut verwendet wird.

7. Kosmetische Verwendung gemäß einem der vorhergehenden Ansprüche, wobei der Extrakt von *Anigozanthos flavidus* aus einem oberirdischen Teil von *Anigozanthos flavidus,* ausgewählt aus Blüten, Blättern, Stängeln und Kombinationen davon, erhalten wird.

8. Kosmetische Verwendung gemäß einem der vorhergehenden Ansprüche, wobei der Extrakt von *Anigozanthos flavidus* mittels Extraktion mit einem polaren Lösemittel erhalten wird.

9. Kosmetische Verwendung gemäß einem der Ansprüche 3 bis 8, wobei die kosmetische Zusammensetzung außerdem wenigstens ein weiteres kosmetisches Mittel enthält, vorzugsweise ausgewählt aus der Gruppe, bestehend aus Vitaminen, Licht- und Sonnenschutzmitteln, Anti-Aging-Mitteln oder Antifaltenmitteln, Antioxidantien, Liftingmitteln, Straffungsmitteln, Mitteln gegen Pigmentflecken, Mitteln gegen Rötungen, Schlankheitsmitteln, Entwässerungsmitteln, Hydratisierungsmitteln, Beruhigungsmitteln, Peeling- oder Scheuermitteln, mattierenden Mitteln, talgregulierenden Mitteln, hautaufhellenden Wirkstoffen, Selbstbräunungsmitteln, Bräunungsbeschleunigern und Kombinationen davon.

10. Kosmetische Verwendung gemäß einem der Ansprüche 3 bis 9, wobei die kosmetische Zusammensetzung aus der Gruppe ausgewählt ist, bestehend aus wässrigen Lösungen, wässrig-alkoholischen Lösungen, Öl-in-Wasser-Emulsionen (O/W) oder Wasser-in-Öl-Emulsionen (W/O) oder mehrfachen (dreifachen: W/O/W oder O/W/O) Emulsionen, Nanoemulsionen, insbesondere W/O-Nanoemulsionen mit einer Tropfengröße kleiner 100 nm, wässrigen Gelen oder Dispersionen einer Fettphase in einer Wasserphase mithilfe von Kügelchen, Suspensionen, vorzugsweise in wässrigen oder wässrig-alkoholischen Medien, Liposomen-Suspensionen, Pulvern, Lotionen, Milchen, Cremes, Salben, Gelen, Schäumen und Pomaden.

11. Kosmetische Verwendung gemäß einem der Ansprüche 3 bis 10, wobei die kosmetische Zusammensetzung aus einem kosmetischen Produkt, einem Schminkprodukt oder einem Körperpflegeprodukt, zum Beispiel einer Lotion, einer Milch, einem Serum, einem öligen oder wässrigen Gel, einer Emulsion, einer Creme, insbesondere einer Tages- oder Nachtcreme, einer Gelcreme, einem Hautpflegewasser, einer Salbe, einem Balsam, einer Foundation, einem Spray, einem Lidschatten, einem Stift, einem Lippenstift, einem Gloss, einem Lippenbalsam, einem Schaum, einem Deodorant, einer Pflegemaske, einem Duschgel und einem Peeling- oder Scheuermittel, ausgewählt ist.

12. Zusammensetzung zur Herstellung einer kosmetischen Zusammensetzung zur Behandlung oder Prävention eines Anzeichens der Alterung, umfassend
- 20 Gew.-% bis 40 Gew.-% eines flüssigen Extrakts, der aus einem oberirdischen Teil von *Anigozanthos flavidus* erhalten ist, als Anti-Aging-Mittel, und
- 60 Gew.-% bis 80 Gew.-% Glycerol.

## Claims

1. Cosmetic use of an extract obtained from an aerial part of *Anigozanthos flavidus* as an anti-ageing agent.

2. The cosmetic use of an extract of *Anigozanthos flavidus* according to claim 1, as an antiwrinkle, smoothing, toning, lifting or relifting, restructuring or remodeling agent of the skin.

3. The cosmetic use according to any one of the preceding claims, wherein the extract of *Anigozanthos flavidus* is present as an active agent in a cosmetic composition intended for preventing, delaying and/or treating one or more signs of skin ageing.

4. The cosmetic use according to claim 3, wherein the sign(s) of skin ageing are selected from the group consisting of: the appearance of fine lines and/or wrinkles on the skin, in particular at the lips and on the eyelids, withering or sagging of the skin, in particular on the neck, a loss of radiance of the skin, a dull complexion, pigment spots, dark circles under the eyes, a loss of density of the skin, a loss of firmness of the skin, a loss of tonicity of the skin, a loss of elasticity of the skin, a loss of contractility of the skin, a loss of maintenance of the skin, a slackening of the skin, an alteration of the smooth appearance of the skin, an increase in the roughness of the skin, and an alteration of the outline of the face.

5. The cosmetic use according to any one of claims 1 to 4, wherein the extract of *Anigozanthos flavidus* is used for improving, restoring or preventing the alteration of mechanical properties of the skin in a mature skin.

6. The cosmetic use according to any one of claims 1 to 4, wherein the extract of *Anigozanthos flavidus* is used for improving the complexion or the radiance of a mature skin.

7. The cosmetic use according to any one of the preceding claims, wherein the extract is obtained from an aerial part of *Anigozanthus flavidus* selected from the flowers, the leaves, the stems and combinations thereof.

8. The cosmetic use according to any one of the preceding claims, wherein the extract of *Anigozanthos flavidus* is obtained by extraction with a polar solvent.

9. The cosmetic use according to any one of claims 3 to 8, wherein the cosmetic composition also comprises at least one additional cosmetic agent, preferably selected from the group consisting of vitamins, sun filters and sunscreens, anti-ageing or antiwrinkle agents, antioxidants, lifting agents, firming agents, anti-spot agents, anti-redness agents, slimming agents, draining agents, moisturizing agents, soothing agents, scrubbing or exfoliating agents, matting agents, sebum regulators, lightening active agents, self-tanning active agents, bronzing accelerators and combinations thereof.

10. The cosmetic use according to any one of claims 3 to 9, wherein the cosmetic composition is selected from the group consisting of aqueous solutions, aqueous-alcoholic solutions, oil-in-water (O/W) or water-in-oil (W/O) or multiple (triple: W/O/W or O/W/O) emulsions, nanoemulsions, in particular O/W nanoemulsions, with a drop size of less than 100 nm, aqueous gels, or dispersions of a fatty phase in an aqueous phase by means of spherules, suspensions, preferably in aqueous or aqueous-alcoholic media, liposome suspensions, powders, lotions, milks, creams, salves, gels, foams and ointments.

11. The cosmetic use according to any one of claims 3 to 10, wherein the cosmetic composition is selected from a cosmetic product, a makeup product or a body hygiene product, for example a lotion, a milk, a serum, an aqueous or oily gel, an emulsion, a cream, in particular a day cream or a night cream, a cream-gel, a skincare water, an ointment, a balm, a foundation, a spray, an eyeshadow, a stick, a lipstick, a gloss, a lip balm, a foam, a deodorant, a nourishing mask, a shower gel, and an exfoliating or scrubbing product.

12. A composition for the preparation of a cosmetic composition intended for treating or preventing skin aging comprising :
- from 20% to 40% by weight of a liquid extract obtained from an aerial part of *Anigozanthos flavidus* as antiaging agent and
- from 60% to 80% by weight of glycerol.
